# EUROPEAN PATENT SPECIFICATION

(11) **EP 2 166 083 B1**
(45) Date of publication and mention of the grant of the patent: **22.12.2010**
(21) Application number: 08173132.5
(22) Date of filing: 31.12.2008
(51) Int. Cl.: C12N 1/20

(54) **Novel Lactobacillus paracasei subsp. paracasei SG96, a bacteriostatic composition containing the same and use thereof**
Neues Lactobacillus paracasei subsp. paracasei SG96, eine bakteriostatische Zusammensetzung damit und Verwendung davon
Nouveau Lactobacillus paracasei subsp. paracasei SG96, composition bactériostatique le contenant et son utilisation

(43) Date of publication of application: 24.03.2010
(73) Proprietor: Syngen biotech CO., LTD., Sinying City (TW)
(72) Inventor: Wei, Yu-Shan, Houbi Shiang (TW); Tai, Fang-Yun, Kaohsiung City (TW)
(74) Representative: Viering, Jentschura & Partner

(56) References cited:
- EP-A- 1 034 787
- WO-A-02/38103
- WO-A-02/053706
- DELGADO S ET AL: "Subtractive screening for probiotic properties of Lactobacillus species from the human gastrointestinal tract in the search for new probiotics" JOURNAL OF FOOD SCIENCE, vol. 72, no. 8, October 2007 (2007-10), pages M310-M315, XP002517362 ISSN: 0022-1147
- TSAI C C ET AL: "Three Lactobacillus strains from healthy infant stool inhibit enterotoxigenic Escherichia coli grown in vitro" ANAEROBE, LONDON, GB, vol. 14, no. 2, 1 April 2008 (2008-04-01), pages 61-67, XP022621240 ISSN: 1075-9964 [retrieved on 2007-12-04]
- WARD L J H ET AL: "Differentiation of Lactobacillus casei, Lactobacillus paracasei and Lactobacillus rhamnosus by polymerase chain reaction" LETTERS IN APPLIED MICROBIOLOGY, vol. 29, no. 2, August 1999 (1999-08), pages 90-92, XP002517363 ISSN: 0266-8254

## Description

### BACKGROUND OF THE INVENTION

### 1. Field of the invention

The invention relates to a novel *Lactobacillus paracasei* subsp. *paracasei* SG96 strain, and in particular, to a novel *Lactobacillus paracasei* subsp. *paracasei* microbial strain that can inhibits pathogenic bacteria such as *Escherichia coli, Salmonella typhimurium,* and the like, a bacteriostatic composition containing said *Lactobacillus paracasei* subsp. *Paracasei* and use of said composition.

### 2. Description of the prior art

In past several decades, since the population increased fast and the level of material life was promoted largely, people's demand for meat products was increased greatly. In order to satisfy market demand, the raising of numerous economic animals has been scaled up gradually. Especially, in backward countries, because their abilities for importing foods were low, economic animals were mass raised in high density or big area. This situation resulted in rapid dissemination of a number of diseases among animals. For solving the sickening trouble of economic animals, or for the fast growth of economic animals, approaches adopted for a long time period were addition of antibiotics in animal feeds, or administration of antibiotics into animals, these had leaded to generation of tremendous drug-resistant strains.

In late 1960 decade, the introduction of antibiotics had caused the humanity to obtain the unprecedented victory in the field of treating bacterial infection. A number of originally fatal infectious diseases became no longer fearful. However, the emergence of drug-resistant bacteria in recent years had elevated once more the anxiety and fear of mankind to infectious diseases, since antibiotics became no more a catholicon for controlling diseases. Further, the dissemination speed of drug-resistant bacteria had surpassed our imagination.

Bacterial drug-resistance brought out not only lowering of treating effect or even ineffectiveness, but also serious influence on the human health. Forty years ago, there were about 7 millions of mankind died in the infectious diseases worldwide every year, while at present, in spite of the great advance in medical science, the annual death due to infectious diseases is increased dramatically to 20 millions. As people takes in long term residual antibiotics-containing food products such as meats, eggs, milks and the like derived from poultry and livestock, these residual antibiotics incur adverse effect on human body, and may store up in the body so as to cause unceasing occurrence of drug-resistant strain.

In recent ten years, safety issue of meat products has accepted most concern by consumers, producers in agriculture, fishing and livestock, as well as government. The constant high concern on meat products by consumers come from several terrified safety events about meat products in domestic and foreign countries in last several years, such as mad cow disease (Bovine Spongiform Encephalopathy) (BSE) in England, H5N1 avian flu in southeast Asia and mainland China and the like. In consideration of the safety in taking meat products, worldwide had paid much attention to the adverse consequences caused by residual antibiotics. In order to delay the occurrence of drug-resistance strain issues, in mid-1990, some European countries had started to restrain and control the use of antibiotics in medicine and agriculture, so as to hope for lowering the generation rate of drug-resistance strain. Since 1995, European Union had forbidden successively the use of some antibiotics in feeds. These approaches had gained good results, for example, researchers in Danish Veterinary Laboratory, Copenhagen indicated that after forbidding the use of avoparcin antibiotic, the drug-resistance to avoparcin by Enterococcus faecium in digestive tract of Danish tender chicken had reduced from 73% in 1995 down to 6% in 2000. After forbidden the use of avoparcin in 5 years of the same period, Germany and Holland had demonstrated that drug-resistance strains in animal and human body had reduced. Consequently, European Union had forbidden the addition of antibiotics in feeds since January 2006.

The purpose of adding antibiotics in feeds is to promote growth, reduce coarse feed, and enhance growth efficiency of poultry and livestock. However, since the vegetative cycle of the poultry and livestock is shorten, the precipitation of nutritive substances is reduced inevitably. Consequently, the unique flavor and quality of the poultry and livestock products will lower, and more seriously, residual drugs brought serious hazard on human health, and especially, on the health of children in growth period. Therefore, forbidding the use of antibiotics or gradual reducing the types and dosages of antibiotics in economic animal feeds becomes a worldwide trend. However, stepwise forbidding of antibiotics may derive many problems, and for giving the animal husbandry normal production, the producer has accepted the concept of using probiotic agent as the substitute for antibiotics and further as a feed additives.

Probiotics are predominantly lactic acid bacteria, because lactic acid bacteria can maintain and restore the balance of bacterial colonies in the digestive tract of poultry and livestock, smooth away the stress of poultry and livestock, enhance the immunological and disease-resistant abilities of poultry and livestock, and decrease morbidity. Furthermore, silage obtained from lactic acid bacteria fermentation, and liquid fermented feed are advocated, since lactic acid bacteria fermented feed not only can retain effectively the nutritive ingredients in the feed and raise rates of digestion and utilization, but also can, during digestion in intestinal tract, eliminate pathogenic bacteria as well as kill certain microorganisms that may induce diseases both in mankind and animal. Lactic acid bacteria had been acknowledged generally as a very ideal substitute for antibiotics.

Beneficial effects of probiotics in the bodies of poultry and livestock include:
1. Maintaining and restoring the balance of bacterial colonies taking lactic acid bacteria as a superiority bacterial colony in the digestive tract of poultry and livestock; Smoothing away the stress state of poultry and livestock (stress, such as weaning, changing feed, high temperature, chill, transportation, frightening, disease and the like), and further eliminating the environmental disorder in the digestive tract; suppressing the abnormal proliferation of harmful bacteria as well as the infiltration and anchoring of pathogenic bacteria; Preventing and treating diseases in digestive tract such as scours, diarrhea, and the like; reducing or eliminating the generation of endotoxin; and especially remarkable, the effect of supplementing lactic acid bacteria for young animal and poultry that have not established their microorganism system.
2. Producing non-specific immunological regulatory factor; enhancing the immunological disease-resistant ability; decreasing disease infection rate and death rate; replacing effectively antibiotics and chemical drugs; reducing residual probability of drugs; and promoting food quality for mankind.
3. Supplementing nutritive ingredients; promoting growth of poultry and livestock; inducing the generation of digestive enzymes; enhancing abilities of digestion and absorption; and increasing remarkably the output and benefit for the husbandry.
4. Reducing odorous substances in the excrements of poultry and livestock; and improving the environment of the farm.
5. Increasing remarkably the output of milk, meat and egg; and adding the benefit of the husbandry.

Safety problem of food products has received high attention from various governments worldwide. At present, governments are promoting positively the green animal husbandry and supplying the development of green products in the policy, the financial resource, and the physical resource. There is a need to build up demonstration base for green farming, to develop a brand of green livestock products, to give operator of green products a material benefit, and to supply consumer high quality, non-industrial nuisance, safe and green poultry and livestock food products so as to benefit the humanity.

### SUMMARY OF THE INVENTION

One object of the invention is to provide a novel *Lactobacillus paracasei* subsp. *paracasei* SG96 strain, said strain is different significantly in phylogenetic relation from conventional *Lactobacillus paracasei* BCRC 910220, *Lactobacillus paracasei* subsp. *paracasei* BCRC14001, and *Lactobacillus paracasei* subsp. *paracasei* BCRC16100, and hence is a novel *Lactobacillus paracasei* subsp. *paracasei* microbial strain.

Another object of the invention is to provide a bacteriostatic composition containing said novel *Lactobacillus paracasei* subsp. *paracasei* SG96, characterized in that said composition has an effect of inhibiting the growth of pathogenic bacteria such as *Escherichia coli, Salmonella typhimurium* and the like.

Yet another object of the invention is to provide the use of said bacteriostatic composition containing said novel *Lactobacillus paracasei* subsp. *paracasei* SG96, characterized in that said bacteriostatic composition can be used as an animal feed, and as additives in drinking water, to enhance the pathogenic bacteria-resistant ability of animal; and that said bacteriostatic composition can be used further as human food products, and as additives in beverages or food products, so as to enhance the pathogenic bacteria-resistant ability in human body.

The novel *Lactobacillus paracasei* subsp. *paracasei* SG96, a bacteriostatic composition containing the same and use thereof that can achieve those above-described objects of the invention comprise: a novel *Lactobacillus paracasei* subsp. *paracasei SG96* strain, said strain is obtained by screening from pig intestinal tract excreta, and after identified through Gram stain analysis, API 50 CHL analysis, 16S rDNA sequencing analysis, and phylogenetic analysis, it is confirmed to be a novel *Lactobacillus paracasei* subsp. *paracasei* microbial strain. Said *Lactobacillus paracasei* subsp. *paracasei* SG96 strain has been deposited in China General Microbiological Culture Collection Center (CGMCC), with accession number as CGMCC 2697, at October 9, 2008.

As *Lactobacillus paracasei* subsp. *paracasei* SG96 strain according to the invention is subjected to disc-agar diffusion analysis and mixed culture experiment analysis with pathogenic bacteria, respectively, results indicated that the inventive SG96 strain can suppress the growth of pathogenic bacteria such as *E. coli, S.typhimurium* and the like, and even has an effect of killing bacteria.

When the inventive *Lactobacillus paracasei* subsp. *paracasei* SG96 strain is formulated into aqueous solution and fed to the un-weaned piglet, the result indicated that the inventive SG96 strain can promote the weight gain of the un-weaned piglet, improve the feed conversion rate of the piglet feed, have good anti-diarrhea effect, and exhibit a performance better than that of conventional antibiotics, and demonstrated that the inventive SG96 strain can enhance pathogenic bacteria-resistant ability of the animal.

In addition to be formulated into aqueous solution to be used as drinking water for animals, the anti-bacteria composition containing the inventive *Lactobacillus paracasei* subsp. *paracasei* SG96 strain can be used in conjunction with conventional feed additive preparation technique and formulated into other forms, including animal feed additives, medical composition for animal and mankind, additive for food product, additive for beverages, as food product, beverage, and healthy food product and the like, for oral administration by animal and mankind. In a preferred embodiment, said anti-bacteria composition can be processed into lactic acid tablet, powder or granules, and can be added into animal feed or food products (for example: 1 Kg of powder or granular *Lactobacillus paracasei* subsp. *paracasei* SG96 strain in 1 ton of feed (10⁷CFU/g)); or can be used alone or in combination with other lactic acid bacteria to ferment milks into milk products such as yogurt milk or yogurt.

These features and advantages of the present invention will be fully understood and appreciated from the following detailed description of the accompanying Drawings.

### BRIEF DESCRIPTION OF THE DRAWINGS

The patent or application file contains at least one drawing executed in color. Copies of this patent or patent application publication with color drawing(s) will be provided by the Office upon request and payment of the necessary fee.
Figure 1A is the Gram stain results of the inventive *Lactobacillus paracasei* subsp. *paracasei* SG96 strain; Figure 1B is the morphology graph of the inventive *Lactobacillus paracasei* subsp. *paracasei* SG96 strain;
Figure 2 is an agarose gel electrophoresis pattern of a PCR-amplified 16S rDNA fragment of said *Lactobacillus paracasei* subsp. *paracasei* SG96 strain;
Figure 3 is a sequence alignment chart of 16S rDNA among *Lactobacillus paracasei* subsp. *paracasei* strain SG96, BCRC 910220, BCRC14001, and BCRC16100;
Figure 4 is a phylogenetic analysis chart of *Lactobacillus paracasei* subsp. *paracasei* strain SG96, BCRC 910220, BCRC14001, BCRC16100 and other *Lactobacillus paracasei* subsp. *paracasei* strain;
Figure 5 shows the inhibition effect of *Lactobacillus paracasei* subsp. *paracasei* SG96 strain on *Escherichia coli* by using disc-agar diffusion analysis;
Figure 6 shows the inhibition effect of *Lactobacillus paracasei* subsp. *paracasei* SG96 strain on *Salmonella typhimurium* by using disc-agar diffusion analysis;
Figure 7 shows the result of mixed culture experiment of *Lactobacillus paracasei* subsp. *paracasei* SG96 strain (10⁸ bacteria count) with pathogenic bacteria;
Figure 8 shows the result of mixed culture experiment of *Lactobacillus paracasei* subsp. *paracasei* SG96 strain (10⁷ bacteria count) with pathogenic bacteria;
Figure 9 shows the result of mixed culture experiment of *Lactobacillus paracasei* subsp. *paracasei* SG96 strain (10⁶ bacteria count) with pathogenic bacteria;
Figure 10 shows the result of mixed culture experiment of *Lactobacillus paracasei* subsp. *paracasei* SG96 strain (10⁵bacteria count) with pathogenic bacteria;
Figure 11 shows the result of mixed culture experiment of *Lactobacillus paracasei* subsp. *paracasei* SG96 strain (10⁶⁴bacteria count) with pathogenic bacteria;
Figure 12 shows the effect of *Lactobacillus paracasei* subsp. *paracasei* SG96 strain on the weight change of un-weaned piglet; and
Figure 13 shows the effect of *Lactobacillus paracasei* subsp. *paracasei* SG96 strain on the diarrhea of un-weaned piglet.

### DETAILED DESCRIPTION OF THE PREFERRED EMBODIMENT

The invention will be illustrated by way of the following examples, but the invention will not be limited thereto.

### Example 1 The screening and isolation of Lactobacillus paracasei subsp. paracasei SG96 strain

Intestinal tract excreta were collected from pigs, placed in MRS broth medium (Difco™, REF288130), and cultured at 37°C under anaerobic condition for 24 hours. Then, the resulted culture suspension was applied over a MRS agar plate (Difco™, REF288210), and incubated at 37°C for 3 days. Thereafter, colonies on the agar medium were collected to obtain 1000 acid forming bacteria strains, which were screened by disc-agar diffusion to obtain lactic acid bacteria having inhibiting activities against *E*. *coli*) (BCRC11634) and *S. typhimurium*) (BCRC129407). A functional *Lactobacillus paracasei* subsp. *paracasei* SG96 having best activity was selected from these lactic acid bacteria. This strain had following culture characteristics: forming a grey colony of 2-3 mm diameter on MRS agar plate, and the best culturing temperature: 37°C. Said *Lactobacillus paracasei* subsp. *paracasei* SG96 had been deposited in China General Microbiological Culture Collection Center (CGMCC), with an accession number: CGMCC 2697, at October 9, 2008.

### Example 2 Gram stain analysis of Lactobacillus paracasei subsp. paracasei SG96 strain

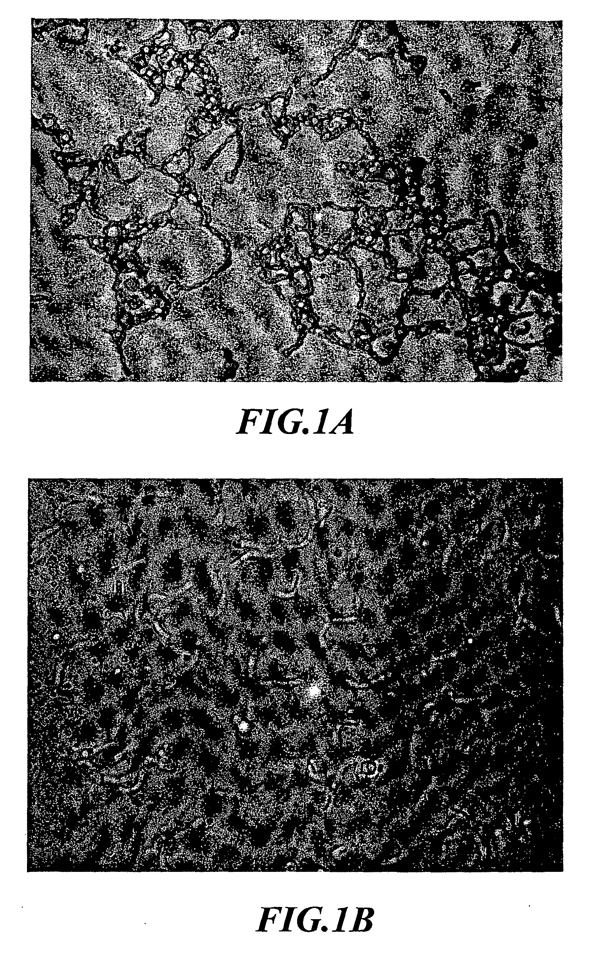

The characteristics of the strain obtained in Example 1 were tested by Gram stain assay. The result was shown in Figure 1A, and revealed that the inventive strain belonged to Gram-positive bacteria, non-sporing anaerobes, and non-mobility bacteria, which were typical characteristics of Lactobacillus.

Said *Lactobacillus paracasei* subsp. *paracasei* SG96 strain has following bacteriological characteristics:
(a) Morphological characteristics:
   (1) Cell shape and size: When the cell was cultured in MRS broth medium at 37°C under anaerobic condition for 24 hours, a bacillus present as rod-shape could be observed under microscopic examination (see Figure 1B).
   (2) Mobility: non-mobility
   (3) Flagellum: none
   (4) Sporogenesis: non-sporing
   (5) Gram stain: positive
(b) Culturing characteristics:
   (1) Medium: MRS broth medium, pH = 6.25
   (2) Culturing condition: 37°C under anaerobic condition
(c) Physiological characteristics:
   (1) Catalase: negative
   (2) Oxidase: negative
   (3) API 50 CHL test: see Example 3.

### Example 3 16S rDNA sequencing analysis of Lactobacillus paracasei subsp. paracasei SG96 strain

### 1. DNA extraction

DNA was purified using FavorPrep™ Blood Genomic DNA Extraction Mimi Kit. The bacteria were cultured overnight. 200 µL of bacteria liquor was mixed with 20 µL proteinase K (proteinase K, before use, 110 µL ddH₂O was added and mixed by shaking for 5 min, to a concentration of 11 mg/mL, and stored at -20 °C till use), together with 200 µL FABG buffer, by shaking for 5 seconds. Then, the mixture was heated at 60°C for 15 minutes and was centrifuged briefly. 200 µL 95% ethanol was added and mixed by shaking for 10 seconds. After brief centrifuge, the mixture was charged into a FABG column. The FABG column was placed in a Collection Tube, and centrifuged at 10,000 rpm for 2 minutes. The FABG column was then removed, the subnatant was decanted and after sucked dried with a tissue paper, the FABG column was returned into the Collection Tube. 500 µL W1 buffer (8mL of 95% ethanol had to be added at the first use) was added, and centrifuged at 10,000 rpm for 2 minutes. Then, the FABG column was removed, and the subnatant was decanted and sucked dried with a tissue paper. The FABG column was returned again in the Collection Tube. 750 µL WB (40mL of 95% ethanol had to be added at the first use) was added, and centrifuged at 10,000 rpm for 2 minutes, and the subnatant was decanted and sucked dried with a tissue paper. The FABG column was returned in the Collection Tube, and centrifuged at 10,000rpm for 6 minutes. Thereafter, the resulting FABG column was placed in a 1.5-ml centrifuge tube. 150 µL of Elution Buffer was added, stood still for 3 minutes, and centrifuged at 10,000 rpm for 4 minutes. A pure DNA was obtained and stored at -20°C till use.

### 2. The amplification of 16S rDNA PCR fragment

DNA of *Lactobacillus paracasei* subsp. *paracasei* SG96 strain, and DNA of *Lactobacillus paracasei* GMNL-32 strain (this strain had an accession number of BCRC 910220, was described in **ROC Patent No.** I284149, and was a *Lactobacillus paracaseis* strain having a function for treating allergy-related condition), were subjected separately to amplification of 16S rDNA (ribosomal DNA) fragment. Primes used were prokaryotic 16S rDNA PCR universal primers that had been designed based on sequences of 8-27 bases and 1510-1492 bases of E. coli 16S rDNA gene **(**William G. Weisburg, Susan M. Barns, Dale A. Pelletier, and David J. Lane. 16S Ribosomal DNA Amplification for Phylogenetic Study. J. Bacteriol. 1991 January; 173(2): 697-703), and had following sequences:
Forward primer fD1:
   5'-AGAGTTTGATCCTGGCTCAG-3' (SEQ ID No: 1)
Reverse primer rP1:
   5'-ACGGTTACCTTGTTACGACTT-3' (SEQ ID No: 2)

Said 16S rDNA PCR fragment amplification was carried out with following process: (1) 95°C 5 minutes; (2) 94°C 1 minute; (3) 60°C 30 seconds; (4) 72°C 1.5 minutes; (5) 72°C 10 minutes, wherein steps (2) to (4) were repeated for 30 cycles.

Further, DNA of *Lactobacillus paracasei* subsp. *paracasei* BCRC14001 and DNA of *Lactobacillus paracasei* subsp. *paracasei* BCRC16100 were subjected separately to amplification of 16S rDNA fragment. Primers used were derived from 16S rRNA V1 region of *Lactobacillus paracasei,* and 16S rRNA conserved sequence of Lactobacillus **(**Ward, L. J. H., and Timmins, M. J. 1999. Differentiation of Lactobacillus casei, Lactobacillus paracasei and Lactobacillus rhamnosus by polymerase chain reaction. Lett. Appl. Microbiol. 29: 90-92**.), and had following sequences:**
Forward primer:
   5'-CACCGAGATTCAACATGG-3' (SEQ ID No: 3)
Reverse primer:
   5'-CCCACTGCTGCCTCCCGTAGGAGT-3' (SEQ ID No: 4)

Said 16S rDNA PCR fragment amplification was carried out with following process: (1) 95°C 5 minutes; (2) 94°C 1 minute; (3) 60°C 30 seconds; (4) 72°C 1.5 minutes; (5) 72°C 10 minutes, wherein steps (2) to (4) were repeated for 30 cycles.

### 3. Sequence alignment of 16S rDNA PCR fragments

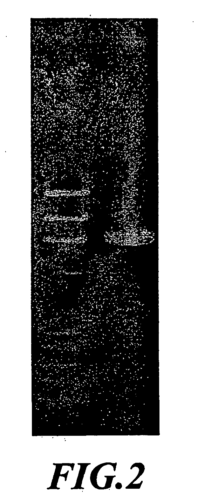

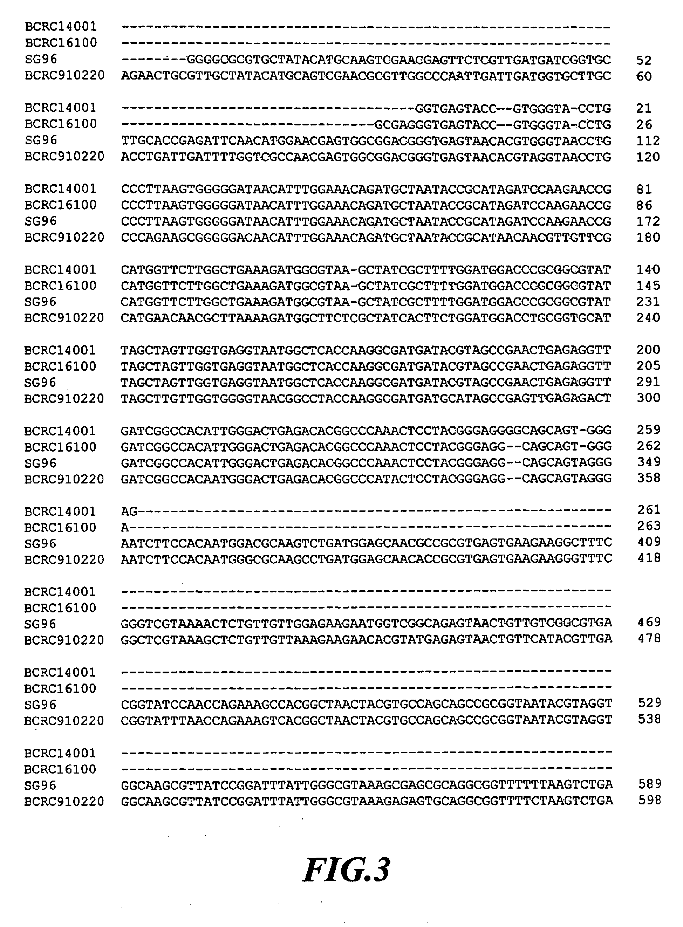

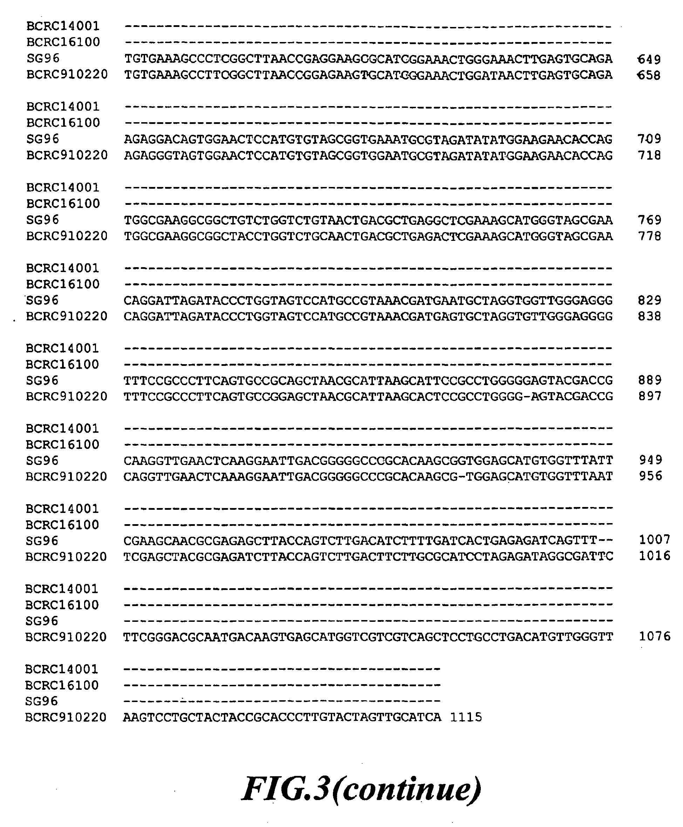

PCR product of 16S rDNA from *Lactobacillus paracasei* subsp. *paracasei* SG96 strain was subjected to agarose gel electrophoresis, with a result shown in Fig. 2. It was known from Fig. 2 that said fragment of PCR product had a size of about 1,500 bp (lengths of fragment standards were in order from top to bottom as follow: 3k, 2k, 1.5k, 1K, 900, 800, 700, 600, 500, 400, 300, 200, 100 bp). In addition, sequencing of the fragment was carried out and obtained the sequence of the 16S rDNA fragment from *Lactobacillus paracasei* subsp. *paracasei* SG96 strain as shown in SEQ ID No: 5. Sequence alignment of said sequence with multiplex sequence alignment data bank (NCBI blastn, http://www.ncbi.nlm.nih.gov/BLAST) was performed, with the results as shown in Figure 3, and indicated that the sequence alignment result was in 98% precision as *Lactobacillus paracasei.*

### Example 4 API 50 CHL analysis of Lactobacillus paracasei subsp. paracasei SG96 strain

API 50 CHL test was used to examine the carbohydrate metabolism performance of the inventive lactic acid bacteria strain. API 50 CHL reagent could be used to identify the difference on Genius or Species among strains. The examination was performed by Food Industry Research and Development Institute, Hsinchu, Taiwan, ROC, and the result of API 50 CHL analysis thus obtained was shown in Table 1 and Appendix 1. The result indicated that there was a homology of 98% in carbohydrate metabolism activities between the inventive strain and *Lactobacillus paracasei* subsp. *paracasei* 1, and accordingly, the API 50 CHL analysis confirmed further that the inventive strain is classified as *Lactobacillus paracasei* subsp. *paracasei.*

**Table 1 Results of API 50 CHL analysis**

| | | | | | | | | | |
|---|---|---|---|---|---|---|---|---|---|
| Reference: 97ID036-L27 | | | | | | | | | |
| Strip: API 50 CHL V5.1 | | | | | | | | | |
| Profile: -----+----+ +++----++- +++++++?-- ++-+-----+ -+------- | | | | | | | | | |
| - | - | - | - | - | + | - | - | - | - |
| 0 | GLY | ERY | DARA | LARA | RIB | DXYL | LXYL | ADO | MDX |
| + | + | + | + | - | - | - | - | + | + |
| GAL | GLU | FRU | MNE | SBE | RHA | DUL | INO | MAN | SOR |
| - | + | + | + | + | + | + | + | ? | - |
| MDM | MDG | NAG | AMY | ARB | ESC | SAL | CEL | MAL | LAC |
| - | + | + | - | + | - | - | - | - | - |
| MEL | SAC | TRE | INU | MLZ | RAF | AMD | GLYG | XLT | GEN |
| + | - | + | - | - | - | - | - | - | - |
| TUR | LYX | TAG | DFUC | LFUC | DARL | LARL | GNT | 2KG | 5KG |
| Significant taxa | | | | | %ID | T | Test against | | |
| *Lactobacillus paracasei subsp. paracasei* 2 | | | | | 98.5 | 0.78 | Gluconic acid (GNT) | | |
| | | | | | | | 83% | | |
| Next taxa | | | | | %ID | T | Test against | | |
| *Lactobacillus paracasei subsp. paracasei* 1 | | | | | 1.4 | 0.55 | Lactose (LAC) 99% | | |
| | | | | | | | Gentiobiose (GEN) 80% | | |
| | | | | | | | Gluconic acid (GNT) 93% | | |

### Example 5 Phylogenetic analysis of Lactobacillus paracasei subsp. paracasei SG96

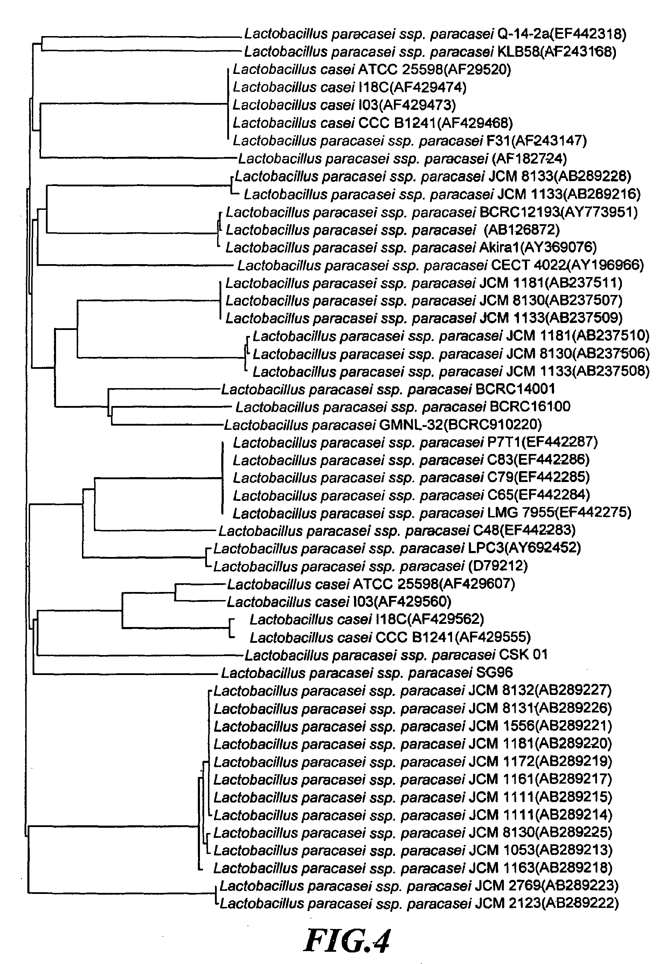

Sequences from 45 *Lactobacillus paracasei* strains, *Lactobacillus paracasei* subsp. *paracasei* and *Lactobacillus casei* published in NCBI Nucleotide Databases (http://www.ncbi.nlm.nih.gov/), sequences from 2 stains of BCRC14001 and BCRC16100 published in BCRC Databases, as well as sequences from each of strains described in ROC Patent and US Patent (GMNL-32 (BCRC910220) and CSK01), were subjected to phylogenetic analysis comparison using EMBL-EBI Clusta1W2 (http://www.ebi.ac.uk/clustalw), and the results of the phylogenetic analysis were shown in Fig. 4. the result indicated that the inventive SG96 belonged to the same clone with only five bacteria strains, and one independent strain could be discriminated from these five strains. This represented that the inventive SG96 had its uniqueness in the classification system, and hence was proven accurately that the inventive SG96 is a novel *Lactobacillus paracasei* subsp. *paracasei* microbial strain.

### Example 6 Inhibition effect of Lactobacillus paracasei subsp. paracasei SG96 strain against Escherichia coli and Salmonella typhimurium

### 1. Disc-agar diffusion assay

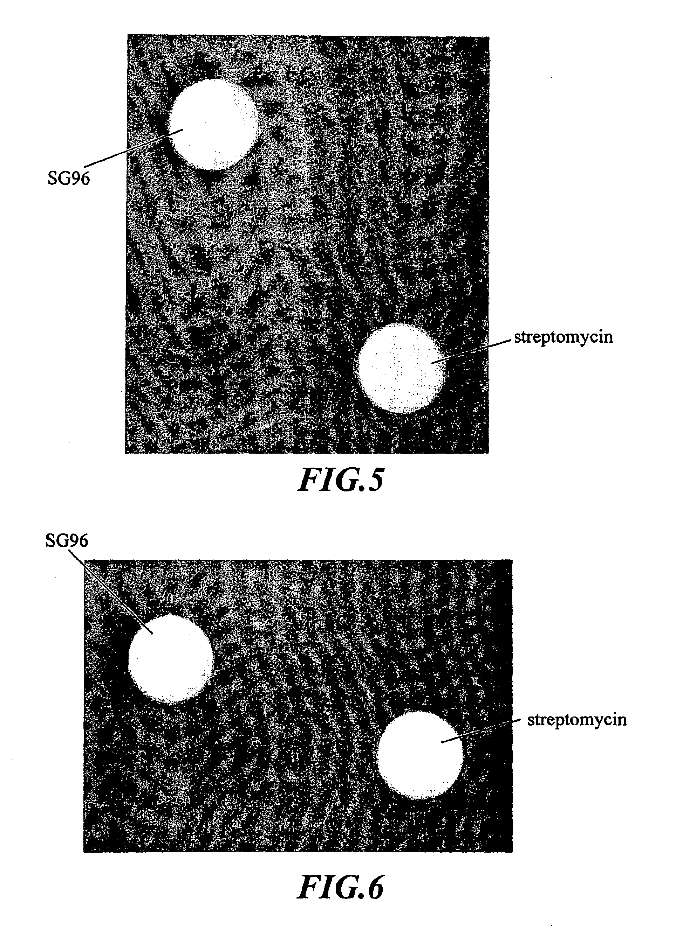

A platinum loop amounts of *Escherichia coli* (accession number: BCRC 11634) and *Salmonella typhimurium* (accession number: BCRC 12947) were inoculated respectively on slants of TSA medium (Soybean-Casein Digest Agar Medium, Difco™ REF236950), and cultured at 35-37°C for 17-24 hours to the stationary growth phase of the strain. After determining the turbidity by a spectrophotometer, the bacteria suspensions were adjusted to a suitable turbidity (a turbidity of 80% at about 600 nm, 80% T). 0.5 ml each of the two pathogenic bacteria liquor was inoculated into a solid plate medium at about 48°C, and the mixture was poured into a Petri dish. After stood still for 1 hour to coagulate, a sterile filter paper tablet of 8 mm in diameter was used to pick up liquor of *Lactobacillus paracasei* subsp. *paracasei* SG96 strain, and the filter paper tablet was placed gently on the above-described Petri dish. After incubated at 35-37°C for 17-24 hours, the result was observed and diameters of the inhibitive circle were measured. 15 g/mL of antibiotic streptomycin was used as the positive control. Results were shown in Fig. 5 and 6.

As shown in Fig. 5 and 6, the diameter of inhibitive circle measured from the inventive *Lactobacillus paracasei* subsp. *paracasei* SG96 strain against *Escherichia coli* (BCRC 11634) was 11.8 mm (see Fig. 5), while the diameter of inhibitive circle from antibiotic streptomycin was 11.0 mm. These indicated that the inventive *Lactobacillus paracasei* subsp. *paracasei* SG96 strain had a stronger inhibitory effect. Further, the diameter of inhibitive circle measured from the inventive strain against *Salmonella typhimurium* (BCRC 12947) was 12.5 mm (see Fig. 6), while the diameter of inhibitive circle of antibiotic streptomycin was 11.2 mm. These indicated also that the inventive *Lactobacillus paracasei* subsp. *paracasei* SG96 strain had a stronger inhibitory effect.

### 2. Mixed culture experiment of Lactobacillus paracasei subsp. paracasei SG96 strain and pathogenic bacteria

In this experiment, *Escherichia coli* (BCRC 11634) and *Salmonella typhimurium* (BCRC 12947) were used as pathogenic bacteria. These pathogenic bacteria were mixed and cultured with *Lactobacillus paracasei* subsp. *paracasei* SG96 strain (TSA medium, at 37°C), and samples were taken during culturing. Samples were diluted serially to a suitable fold and aliquots each of 100 µL were coated separately on MRS and TSA Petri dishes. The MRS Petri dish was incubated at 35-37°C under anaerobic condition for 24-48 hours, while TSA Petri dish was incubated at 35-37°C under aerobic condition for 24 hours. After colonies were formed, bacteria counts of *Lactobacillus paracasei* subsp. *paracasei* SG96 strain (MRS Petri dish) and pathogenic bacteria (TSA Petri dish) were determined.

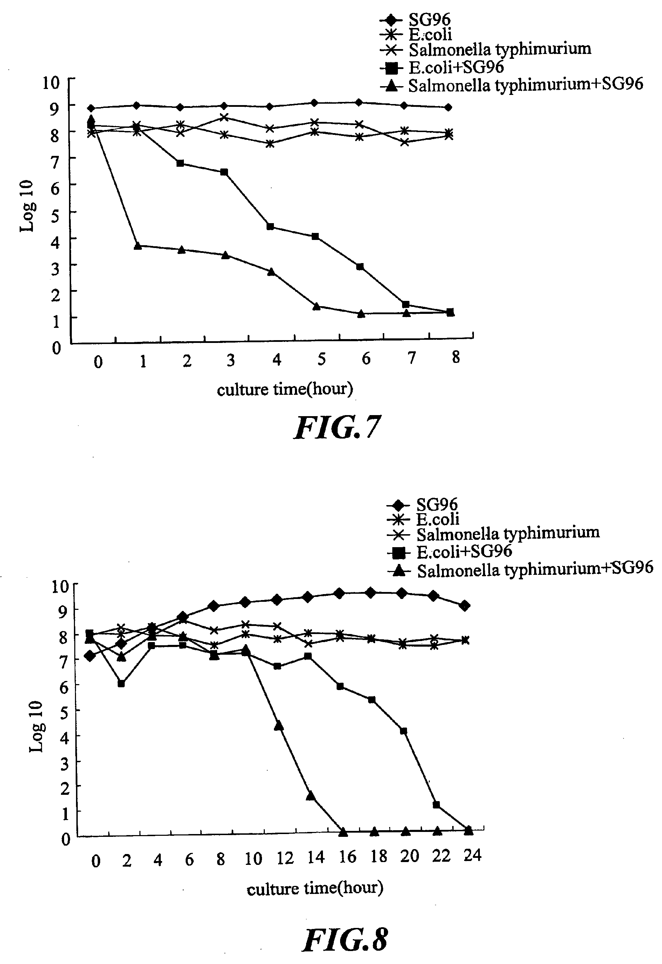

As shown in Fig. 7, when *Lactobacillus paracasei* subsp. *paracasei* SG96 strain (10⁸ CFU/ml) was mixed and cultured with *Escherichia coli* (BCRC 11634) and *Salmonella typhimurium* (BCRC 12947), respectively, the bacteria count of *Escherichia coli* was decreased form 10⁸ to 10¹ within 8 hours, while the bacteria count of *Salmonella typhimurium* was decreased from 10⁸ to 10¹ within 6 hours. These indicated that the inventive strain had a strong inhibitory effect, and even had an effect of killing bacteria.

As shown in Fig. 8, when *Lactobacillus paracasei* subsp. *paracasei* SG96 strain (10⁷ CFU/ml) was mixed and cultured with *Escherichia coli* (BCRC 11634) and *Salmonella typhimurium* (BCRC 12947), respectively, the bacteria count of *Escherichia coli* was decreased form 10⁸ to 0 within 22 hours, while the bacteria count of *Salmonella typhimurium* was decreased from 10⁸ to 0 within 16 hours.

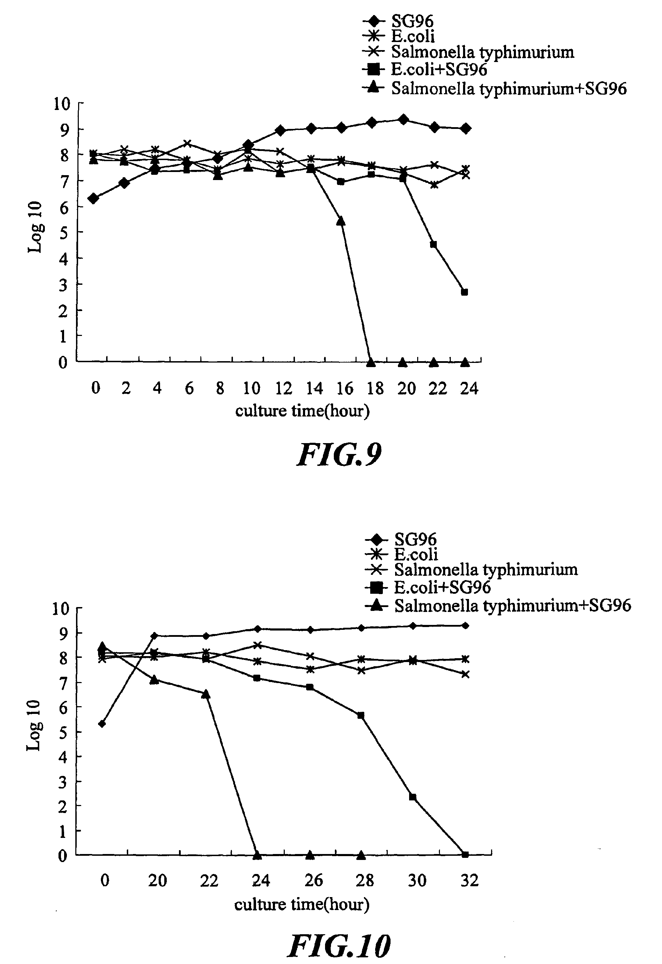

As shown in Fig. 9, when *Lactobacillus paracasei* subsp. *paracasei* SG96 strain (10⁶ CFU/ml) was mixed and cultured with *Escherichia coli* (BCRC 11634) and *Salmonella typhimurium* (BCRC 12947), respectively, the bacteria count of *Escherichia coli* was decreased form 10⁸ to 10² within 24 hours, while the bacteria count of *Salmonella typhimurium* was decreased from 10⁸ to 0 within 18 hours.

As shown in Fig. 10, when *Lactobacillus paracasei* subsp. *paracasei* SG96 strain (10⁵ CFU/ml) was mixed and cultured with *Escherichia coli (BCRC* 11634) and *Salmonella typhimurium* (BCRC 12947), respectively, the bacteria count of *Escherichia coli* was decreased form 10⁸ to 0 after 22 hours, while the bacteria count of *Salmonella typhimurium* was decreased from 10⁸ to 0 within 24 hours.

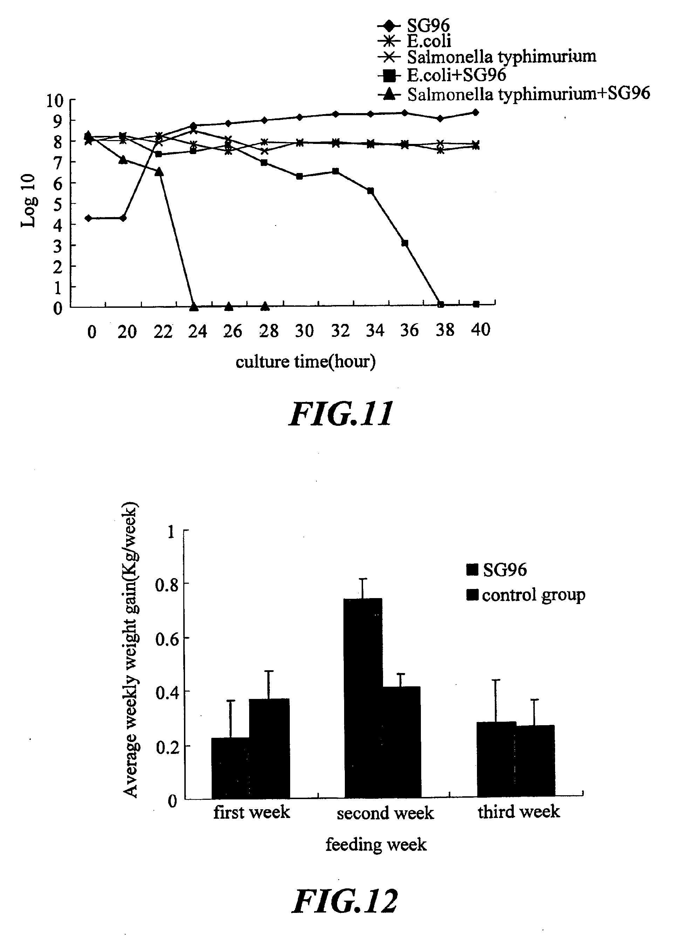

As shown in Fig. 11, when *Lactobacillus paracasei* subsp. *paracasei* SG96 strain (10⁴ CFU/ml) was mixed and cultured with *Escherichia coli (BCRC* 11634) and *Salmonella typhimurium* (BCRC 12947), respectively, the bacteria count of *Escherichia coli* was decreased form 10⁸ to 0 after 38 hours, while the bacteria count of *Salmonella typhimurium* was decreased from 10⁸ to 0 within 24 hours.

### Example 7 Animal test

### 1. The effect of Lactobacillus paracasei subsp. paracasei SG96 strain on the body weight of natal un-weaned piglet

Dairy farmer selected natal un-weaned piglets born from a same sow at the same time, and divided them into two group. One group was treated as a test group and was administrated only with aqueous solution containing 1*10⁷ CFU/mL of *Lactobacillus paracasei* subsp. *paracasei* SG96 strain, and the other group was treated as a control group by feeding antibiotic, oxytetracycline (OTC) alone, for a total feeding time of 21 days. During the period, weight changes of the piglet were recorded every week, and performed statistical analysis by T-test. Result was shown in Fig. 12. After feeding with *Lactobacillus paracasei* subsp. *paracasei* SG96 strain for two weeks, the resulted weight gain rate was more remarkable than that of the control group (p<0.05), wherein the average weekly weight gain of the test group was 0.7-0.8 Kg. After feeding with *Lactobacillus paracasei* subsp. *paracasei* SG96 strain for three weeks, the total weight gain of piglets in the test group was also higher than that of the control group. These indicated that feeding with *Lactobacillus paracasei* subsp. *paracasei* SG96 strain could promote the weight gain of the un-weaned piglet, and improved feed conversion rate of feeds for the piglet.

### 2. The effect of reducing diarrhea for natal un-weaned piglet using Lactobacillus paracasei subsp. paracasei SG96 strain

Since natal un-weaned piglets are susceptible to diarrhea that is commonly caused by sow nursing or environmental factors, piglets dies early often due to serious diarrhea condition, it is desirable for normal growth of piglets if the diarrhea condition can be improved earlier.

Dairy farmer selected natal un-weaned piglets born from a same sow at the same time, and divided them into two groups. One group was treated as a test group and was administrated only with aqueous solution containing 1*10⁷ CFU/mL of *Lactobacillus paracasei* subsp. *paracasei* SG96 strain, and the other group was treated as a control group by feeding antibiotic, oxytetracycline (OTC) alone, for a total feeding time of 21 days. During the period, diarrhea condition of every piglet was recorded every week and gave a corresponding score according to the standard described in the literature published by **Underdahl et al. (**Underdahl NR, Torres-Medina A, Dosten AR. 1982. Effect of Streptococcus faecium C-68 in control of Escherichia coli-induced diarrhea in gnotobiotic pigs. Am. J. Vet. Res. 1982. 12: 2227-2232**).** The score was given as follows:
0 = normal;
1 = soft stool, dry around anus;
2 = moist around anus;
3 = watery diarrhea;
4 = standard 1-3 + bad appetite, weight lost and emaciated.

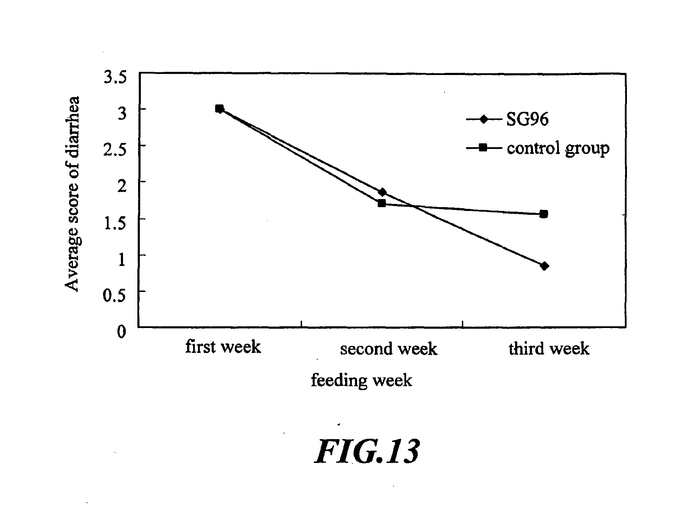

A statistical analysis was performed by T-test, and the result was shown in Fig. 13. Test group fed with *Lactobacillus paracasei* subsp. *paracasei* SG96 strain demonstrated an effect of reducing diarrhea, with the effect similar to that of the control group at the second week. After feeding for three weeks, the effect of reducing diarrhea by feeding with *Lactobacillus paracasei* subsp. *paracasei* SG96 strain was better than that of the control group (the group fed with antibiotics), and differed significantly therebetween (p<0.05). These indicated that the inventive *Lactobacillus paracasei* subsp. *paracasei* SG96 strain exhibited good anti-diarrhea effect, and the performance was better than that obtained from antibiotics commonly used by dairy farmer, as well as could achieve the purpose of leaving no residual antibiotics in the body of the piglet.

In view of the foregoing, the novel *Lactobacillus paracasei* subsp. *paracasei* SG96, a bacteriostatic composition containing the same and use thereof provided by the invention has further following advantages:
1. The inventive *Lactobacillus paracasei* subsp. *paracasei* SG96 strain has been confirmed through Gram stain analysis, API 50 CHL analysis, 16S rDNA sequencing analysis, phylogenetic analysis, to be different in phylogenetic relation from conventional *Lactobacillus paracasei* BCRC 910220, and *Lactobacillus paracasei* subsp. *paracasei* BCRC14001, and BCRC16100, and is a novel *Lactobacillus paracasei* subsp. *paracasei.*
2. When the inventive *Lactobacillus paracasei* subsp. *paracasei* SG96 strain is subjected to disc-agar diffusion analysis and mixed culture experiment analysis with pathogenic bacteria, respectively, results indicate that the inventive SG96 strain not only can inhibit the growth of pathogenic bacteria such as *Escherichia coli, Salmonella typhimurium* and the like, but also its bacteriostatic effect is stronger that that of antibiotic streptomycin, and even has an effect of killing bacteria.
3. When the inventive *Lactobacillus paracasei* subsp. *paracasei* SG96 strain is formulated into aqueous solution and fed to un-weaned piglets, results show that the inventive SG96 strain can promote remarkably the weight gain of un-weaned piglet, improve the feed conversion rate of the feed for the piglet, and has a good anti-diarrhea effect with a performance better than that obtained using conventional antibiotics. These prove that the inventive SG96 strain possesses an ability of enhancing the pathogenic bacteria-resistance of animals.

Many changes and modifications in the above described embodiment of the invention can, of course, be carried out without departing from the scope thereof. Accordingly, to promote the progress in science and the useful arts, the invention is disclosed and is intended to be limited only by the scope of the appended claims.

## Claims

1. A novel *Lactobacillus paracasei* subsp. *paracasei* SG96 strain, which has been deposited in China General Microbiological Culture Collection Center (CGMCC), with accession number: CGMCC 2697.

2. A composition comprising *Lactobacillus paracasei* subsp. *paracasei* SG96 strain of claim 1.

3. A composition as recited in claim 2, wherein said composition is used for inhibiting the growth of *Escherichia coli.*

4. A composition as recited in claim 2, wherein said composition is used for inhibiting the growth of *Salmonella typhimurium.*

5. A composition as recited in claim 2, wherein said composition is used for inhibiting diarrhea.

6. A composition as recited in claim 2, wherein said composition is used as an additive in animal drinking water, as an additive in animal feed, as an medical composition for animal and mankind, as an additive in food product, as an additive in beverage, and as a food product, beverage, and healthy food products.

## Patentansprüche

1. Ein neuer *Lactobacillus paracasei* Unterklasse *paracasei* SG96 Stamm, der im China General Microbiological Culture Collection Center (CGMCC) mit der Zugangsnummer CGMCC 2697 hinterlegt worden ist.

2. Eine Zusammensetzung, die den *Lactobacillus paracasei* Unterklasse *paracasei* SG96 Stamm gemäß Anspruch 1 umfasst.

3. Eine Zusammensetzung gemäß Anspruch 2, wobei die Zusammensetzung zum Hemmen des Wachstums von *Escherichia coli* verwendet wird.

4. Eine Zusammensetzung gemäß Anspruch 2, wobei die Zusammensetzung zum Hemmen des Wachstums von *Salmonella typhimurium* verwendet wird.

5. Eine Zusammensetzung gemäß Anspruch 2, wobei die Zusammensetzung zum Hemmen von Diarrhö verwendet wird.

6. Eine Zusammensetzung gemäß Anspruch 2, wobei die Zusammensetzung als Zusatz in Trinkwasser für Tiere, als Zusatz in Tierfutter, als medizinische Zusammensetzung für Tier oder Mensch, als Zusatz in einem Nahrungsmittelerzeugnis, als Zusatz in einem Getränk und als Nahrungsmittelerzeugnis, Getränk und gesundes Nahrungsmittelerzeugnis verwendet wird.

## Revendications

1. Nouvelle souche de *Lactobacillus paracasei,* sous-espèce *paracasei,* souche SG96, qui a été déposée au Centre général chinois de cultures microbiologiques (CGMCC) sous le numéro d'accès : CGMCC 2697.

2. Composition comprenant une souche de *Lactobacillus paracasei,* sous-espèce *paracasei,* souche SG96 de la revendication 1.

3. Composition telle que décrite dans la revendication 2, où ladite composition est utilisée pour inhiber la croissance *d'Escherichia coli.*

4. Composition telle que décrite dans la revendication 2, où ladite composition est utilisée pour inhiber la croissance de *Salmonella typhimurium.*

5. Composition telle que décrite dans la revendication 2, où ladite composition est utilisée pour inhiber la diarrhée.

6. Composition telle que décrite dans la revendication 2, où ladite composition est utilisée en tant qu'additif dans l'eau potable pour les animaux, en tant qu'additif dans la nourriture animale, en tant que composition médicale pour l'animal et l'être humain, en tant qu'additif dans un produit alimentaire, en tant qu'additif dans des boissons, et en tant que produit alimentaire, boisson, et produits alimentaires bénéfiques pour la santé.


### sequence Listing

<110> Syngen biotech CO., LTD.
<120> Novel Lactobacillus paracasei subsp. paracasei SG96, a Bacteriostatic composition containing the same and use Thereof
<130> P 38980
<140>
   <141> 2008-12-31
<160> 5
<210> 1
   <211> 20
   <212> DNA
   <213> Artificial sequence
   <220>
   <223> Forward primer
<400> 1
   agagtttgat cctggctcag 20
<210> 2
   <211> 21
   <212> DNA
   <213> Artificial sequence
   <220>
   <223> Reverse primer
<400> 2
   acggttacct tgttacgact t 21
<210> 3
   <211> 18
   <212> DNA
   <213> Artificial sequence
   <220>
   <223> Forward primer
<400> 3
   caccgagatt caacatgg 18
<210> 4
   <211> 24
   <212> DNA
   <213> Artificial sequence
   <220>
   <223> Reverse primer
<400> 4
   cccactgctg cctcccgtag gagt 24
<210> 5
   <211> 1007
   <212> DNA
   <213> Lactobacillus paracasei subsp. paracasei SG96 strain
<400> 5
   ggggcgcgtg ctatacatgc aagtcgaacg agttctcgtt gatgatcggt gcttgcaccg  60
   agattcaaca tggaacgagt ggcggacggg tgagtaacac gtgggtaacc tgcccttaag  120
   tgggggataa catttggaaa cagatgctaa taccgcatag atccaagaac cgcatggttc  180
   ttggctgaaa gatggcgtaa gctatcgctt ttggatggac ccgcggcgta ttagctagtt  240
   ggtgaggtaa tggctcacca aggcgatgat acgtagccga actgagaggt tgatcggcca   300
   cattgggact gagacacggc ccaaactcct acgggaggca gcagtaggga atcttccaca   360
   atggacgcaa gtctgatgga gcaacgccgc gtgagtgaag aaggctttcg ggtcgtaaaa   420
   ctctgttgtt ggagaagaat ggtcggcaga gtaactgttg tcggcgtgac ggtatccaac   480
   cagaaagcca cggctaacta cgtgccagca gccgcggtaa tacgtaggtg gcaagcgtta   540
   tccggattta ttgggcgtaa agcgagcgca ggcggttttt taagtctgat gtgaaagccc   600
   tcggcttaac cgaggaagcg catcggaaac tgggaaactt gagtgcagaa gaggacagtg   660
   gaactccatg tgtagcggtg aaatgcgtag atatatggaa gaacaccagt ggcgaaggcg   720
   gctgtctggt ctgtaactga cgctgaggct cgaaagcatg ggtagcgaac aggattagat   780
   accctggtag tccatgccgt aaacgatgaa tgctaggtgg ttgggagggt ttccgccctt   840
   cagtgccgca gctaacgcat taagcattcc gcctggggga gtacgaccgc aaggttgaac   900
   tcaaggaatt gacgggggcc cgcacaagcg gtggagcatg tggtttattc gaagcaacgc   960
   gagagcttac cagtcttgac atcttttgat cactgagaga tcagttt                1007